Europäisches Patentamt

European Patent Office

Office européen des brevets

(19)

Numéro de publication: **0 277 455**
**A1**

## (12) DEMANDE DE BREVET EUROPEEN

(21) Numéro de dépôt: **87480026.1**

(22) Date de dépôt: **22.12.87**

(51) Int. Cl.⁴: **A61K 7/06**

(30) Priorité: **13.01.87 FR 8700358**

(43) Date de publication de la demande:
**10.08.88 Bulletin 88/32**

(84) Etats contractants désignés:
**AT BE CH DE ES GB GR IT LI LU NL SE**

(71) Demandeur: **Errani, Cécile**
**Viale Gozzadini 52**
**I-40000 Bologne(IT)**

(72) Inventeur: **Nogues, Pierre Hôtel Mirabeau**
**1 avenue Princesse Grace**
**Monte Carlo(MC)**

(74) Mandataire: **Hautier, Jean-Louis**
**Cabinet Hautier Office Méditerranéen de Brevets d'Invention et de Marques 24 rue Masséna**
**F-06000 Nice(FR)**

(54) **Composition capillaire.**

(57) L'invention a pour objet une composition capillaire.

La composition capillaire perfectionnée est à base de Centella Asiatica et de dérivés terpéniques. La composition convient au traitement de la chute des cheveux et à l'amélioration de la repousse de ceux-ci.

Composition capillaire pour cheveux.

EP 0 277 455 A1

L'invention a pour objet une composition capillaire. Elle se rapporte plus spécialement à une composition applicable sur le cuir chevelu afin de stopper la chute excessive des cheveux et de permettre leur repousse.

La chute des cheveux, le mauvais aspect de ceux-ci sont extrêmement fréquents cheq l'homme comme chez la femme et présentent une gêne qui va depuis un simple facteur esthétique jusqu'à un trouble psychique. Même les personnes dont l'état général n'est pas mauvais présentent des déficiences capillaires souvent signe avant-coureur d'un alopécie précoce.

L'utilisation de compositions topiques pour le traitement de la chute des cheveux est connue depuis longtemps. Ainsi, trouve-t-on, par exemple, des produits dérivés des cystines, des extraits placentaires, des extraits de plantes, du panténol sous forme liquide ou de pommade.

La présente invention apporte un progrès sensible par l'application de substances qui n'ont pas été proposées pour le traitement de la chute des cheveux chez les personnes présentant un état physique général satisfaisant.

L'application d'une composition à base d'extraits de Centella Asiatica et de dérivés terpéniques, particulièrement les triterpènes permettent :
- de lutter contre la chute des cheveux
- d'activer leur repousse
-d'améliorer l'état de cuir chevelu, particulièrement les états séborrhéiques.

Cette action peut encore être complétée et exaltée par l'adjonction de certaines autres substances actives, ainsi une nouvelle composition, suivant l'invention, par exemple, sera formulée :
Extrait de Centella Asiatica... 10 %
- Essence d'orange douce... 0,5 %
- Crémophor EL... 0,2 %
- Germall 115... 0,3 %
- Eau purifiée q.s.p.... 100 %

Cette composition présente un pH ne dépassant pas 5,5 afin de permettre une meilleure pénétration du produit à travers le cuir chevelu.

Il va de soi que l'on peut modifier certains composants par adjonction de substances actives citées ci-dessus en respectant le noyau de la formule, l'extrait de Centella Asiatica, essence d'orange douce ou autres extraits de plantes contenant une haute teneur en triterpènes.

Cette composition peut se présenter sous forme crème ou gel. A titre d'exemple, nous citerons :
- Essence d'orange douce... 0,5 %
- Cetella Asiatica... 10 %
- Alcool cétylique... 2 %
- Lanette S.X.... 11 %
- Palmitate d'Isopropyle... 5 %
- Huile de vaseline... 6 %

- Parfum... 2 %
- Germall 115... 0,5 %
- Kathon C.G.... 0,13 %
- Eau déminéralisée q.s.p.... 100 %

Pour une efficacité plus grande et plus rapide du produit, des compositions peuvent être obtenues par l'augmentation de la teneur en dérivés terpéniques : exemple 1 % en poids.

Il est intéressant de noter que grâce à la co-action de l'extrait de Centella Asiatica avec des dérivés terpéniques à doses suffisantes, on arrive à faire exercer une action thérapeutique non seulement au niveau du cuir chevelu mais également au niveau du bulbe pileux.

Cette association permet une action efficace sur la pousse des cheveux par vitalisation de sa racine.

Le traitement s'effectue par application du produit sur le cuir chevelu après avoir effectué un shampooing, un séchage, un massage de drainage du cuir chevelu permettant une meilleure vascularisation pour la pénétration du produit.

## Revendications

1. Composition capillaire convenant au traitement de la chute des cheveux et à l'amélioration de la repousse de ceux-ci-, caractérisée par le fait qu'elle comporte :
- un extrait de Centella Asiatica
- et des dérivés terpéniques.

2. Composition capillaire convenant au traitement de la chute des cheveux et à l'amélioration de la repousse de ceux-ci, selon la revendication 1 caractérisée par le fait que les dérivés terpéniques sont des triterpènes.

3. Composition capillaire convenant au traitement de la chute des cheveux et à l'amélioration de la repousse de ceux-ci, selon la revendication 1 caractérisée par le fait qu'elle comporte :
- un extrait de Centella Asiatica
- des essences d'orange douce.

4. Composition capillaire convenant au traitement de la chute des cheveux et à l'amélioration de la repousse de ceux-ci, selon la revendication 3 caractérisée par le fait qu'elle comporte :
- un extrait de Centella Asiatica
- des extraits de plantes contenant une haute teneur en triterpènes.

5. Composition capillaire convenant au traitement de la chute des cheveux et à l'amélioration de la repousse de ceux-ci, selon la revendication 1 caractérisée par le fait qu'elle contient :
- Extrait de Centella Asiatica... 10 %
- Essence d'orange douce... 0,5 %

- Crémophor EL... 0,2 %
- Germall 115... 0,3 %
- Eau purifiée q.s.p.... 100 %

6. Composition capillaire convenant au traitement de la chute des cheveux et à l'amélioration de la repousse de ceux-ci, selon l'une quelconque des revendications 1 ou 5 caractérisée par le fait que cette composition présente un pH ne dépassant pas 5,5 afin de permettre une meilleure pénétration du produit à travers le cuir chevelu.

7. Composition capillaire convenant au traitement de la chute des cheveux et à l'amélioration de la repousse de ceux-ci, selon la revendication 1 caractérisée par le fait qu'elle peut se présenter sous la forme d'un gel dont la formule peut être la suivante :
- Essence d'orange douce... 0,5 %
- Centella Asiatica... 10 %
- Alcool cétylique... 2 %
- Lanette S.X.... 11 %
- Palmitate d'Isopropyle... 5 %
- Huile de vaseline... 6 %
- Parfum... 2 %
- Germall 115... 0,5 %
- Kathon C.G.... 0,13 %
- Eau déminéralisée q.s.p.... 100 %

8. Composition capillaire convenant au traitement de la chute des cheveux et à l'amélioration de la repousse de ceux-ci, selon l'une quelconque des revendications 1, 2, 3, 4, 5, 6 ou 7 caractérisée par le fait que la teneur en dérivés terpéniques peut être augmentée jusqu'à 1 % en poids.

Office européen
des brevets

**RAPPORT DE RECHERCHE EUROPEENNE**

Numero de la demande

EP  87 48 0026

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 022 046  (ETABLISSEMENT RINRONE) <br> * En entier * <br> --- | 1,2,4,8 | A 61 K   7/06 |
| A | FR-A-2 413 093  (CYTHO S.A.R.L.) <br> * En entier * <br> --- | 1,2,4,8 | |
| A | CHEMICAL ABSTRACTS, vol. 101, no. 8, août 1985, page 296, résumé no. 59990e, Columbus, Ohio, US; & JP-A-59 88 412 (LION CORP.) 22-05-1984 <br> --- | 1,4 | |
| A | FR-A-2 191 878  (TOUZET) <br> * Revendications 1,2,4,8 * <br> ----- | 1-8 | |

DOMAINES TECHNIQUES RECHERCHES (Int. Cl.4)

A 61 K

Le présent rapport a été établi pour toutes les revendications

| Lieu de la recherche | Date d'achèvement de la recherche | Examinateur |
|---|---|---|
| LA HAYE | 21-04-1988 | FISCHER J.P. |